(19) Europäisches Patentamt

European Patent Office

Office européen des brevets

(11) **EP 0 864 092 B1**

(12) **EUROPÄISCHE PATENTSCHRIFT**

(45) Veröffentlichungstag und Bekanntmachung des Hinweises auf die Patenterteilung:
**07.11.2001 Patentblatt 2001/45**

(51) Int Cl.[7]: **G01N 33/86**

(21) Anmeldenummer: **96927067.7**

(22) Anmeldetag: **01.08.1996**

(86) Internationale Anmeldenummer:
**PCT/EP96/03383**

(87) Internationale Veröffentlichungsnummer:
**WO 97/10509 (20.03.1997 Gazette 1997/13)**

(54) **KOMPLEX-GEBUNDENE HEMMSTOFFE AKTIVIERBARER STOFFWECHSELENZYME ALS MOLEKULARE MARKER ZUR DIAGNOSTIK UND THERAPIEÜBERWACHUNG**

COMPLEX-BOUND INHIBITORS OF METABOLIC ENZYMES CAPABLE OF BEING ACTIVATED, USEFUL AS MOLECULAR MARKERS FOR DIAGNOSTIC AND THERAPY MONITORING PURPOSES

INHIBITEURS D'ENZYMES METABOLIQUES ACTIVABLES LIES A DES COMPLEXES, UTILISES COMME MARQUEURS MOLECULAIRES POUR LE DIAGNOSTIC ET LE CONTROLE THERAPEUTIQUE

(84) Benannte Vertragsstaaten:
**AT BE CH DE DK ES FR GB IE IT LI NL SE**

(30) Priorität: **13.09.1995 DE 19533817**

(43) Veröffentlichungstag der Anmeldung:
**16.09.1998 Patentblatt 1998/38**

(73) Patentinhaber: **Max-Planck-Gesellschaft zur Förderung der Wissenschaften e.V.**
**80539 München (DE)**

(72) Erfinder:
• **NOWAK, Götz**
  **D-99097 Erfurt (DE)**
• **BUCHA, Elke**
  **D-99094 Erfurt (DE)**
• **BALDINGER, Verena**
  **D-63546 Hammersbach (DE)**

(74) Vertreter: **Weisert, Annekäte, Dipl.-Ing. Dr.-Ing.**
**Patentanwälte**
**Kraus & Weisert**
**Thomas-Wimmer-Ring 15**
**80539 München (DE)**

(56) Entgegenhaltungen:
**EP-A- 0 345 616          EP-A- 0 557 199**
**WO-A-92/05749**

• **PATENT ABSTRACTS OF JAPAN vol. 012, no. 496 (C-555), 23.Dezember 1988 & JP 63 209587 A (FUJI OIL CO LTD), 31.August 1988,**
• **DATABASE WPI Section Ch, Week 7630 Derwent Publications Ltd., London, GB; Class B04, AN 76-57141X XP002022851 & JP 51 020 597 B (GREEN CROSS CORP) , 26.Juni 1976**

## Beschreibung

Die Erfindung betrifft die Verwendung eines an einen großmolekularen Träger gebundenen Hemmstoffs eines Aktivierungsprodukts der Blutgerinnungskaskade oder eines aktivierten Enzyms der Fibrinolyse als molekularer Marker zur Herstellung eines Präparats zur Bestimmung einer Aktivierung dieses Enzyms zur Diagnostik. Bevorzugt betrifft die Erfindung die Verwendung eines an einen großmolekularen Träger gebundenen Thrombininhibitors als molekularen Marker zur Bestimmung einer Gerinnungsaktivierung bei der Gerinnungsdiagnostik und Therapieüberwachung. Insbesondere betrifft die Erfindung Komplex-gebundenes Hirudin (CBH) als molekularer Marker zur Bestimmung einer Gerinnungsaktivierung.

Viele stoffwechselphysiologische Vorgänge werden über oft verzweigte Kaskadenmechanismen reguliert, bei denen über eine Reihe von aktivierbaren Enzymen ein Auslöser oder eine Kettenreaktion verstärkt wird. Solche Mechanismen sind beispielsweise bei der Regulation des Glykogenstoffwechsels, bei der Übertragung extrazellulärer Signale und insbesondere bei der Blutgerinnung wirksam. Auf molekularer Ebene findet man hierbei oft eine sequenzielle Phosphorylierung der an einem Kaskadenmechanismus beteiligten Faktoren. Die Verstärkung eines Auslösers durch diesen Mechanismus beruht dabei auf der Fähigkeit der an den unterschiedlichen Stufen des Mechanismus beteiligten Enzyme, jeweils mehrere Substratmoleküle, die selbst oft ebenfalls wieder Enzyme sind, zu modifizieren. Die aktivierten Enzyme eines solchen Mechanismus eignen sich als molekulare Marker zur Erfassung der jeweiligen im Körper ablaufenden Aktivierungsreaktion. Hierbei werden hochaffine und spezifische Inhibitoren des jeweiligen aktivierten Schlüsselenzyms eingesetzt, die so einer permanenten Diagnostikstrecke zugeführt werden sollen. Dieses sogenannte molekulare Markerprinzip wurde bisher jedoch nur unzureichend entwickelt.

Die Suche nach geeigneten molekularen Markern zur Erfassung von intravasalen Aktivierungsreaktionen der Blutgerinnung wird seit einigen Jahren intensiv vorangetrieben. Dabei haben sich eine Reihe von Stoffwechselprodukten der Gerinnungsaktivierung als molekulare Marker angeboten. Dazu gehören die Prothrombin F1+2-Fragmente, der Plättchenfaktor IV, die Fibrinopeptide A und B, wie auch einige, durch die Fibrinolyse erzeugte, Spaltprodukte des Fibrinabbaus (z.B. d-Dimere), aber auch Komplexe zwischen natürlichen Antithrombinen und der Serinprotease Thrombin, die als TAT-Komplexe bekannt sind.

In großen klinischen Studien wurde die Brauchbarkeit dieser molekularen Marker analysiert. Es konnte generell festgestellt werden, daß durchaus erhöhte bzw. persistierende Blutspiegel von derartigen molekularen Markern bei thrombotischen Ereignissen bzw. thromboembolischen Erkrankungen nachweisbar waren. Die Effizienz dieser Marker ließ jedoch sehr zu wünschen übrig. Bei klinischen Patienten, die an venösen Thrombosen bzw. arteriellen thrombotischen Verschlußerkrankungen litten, ergab sich eine Ansprechbarkeit des empfindlichsten Markers der Gerinnung, des Prothrombin-Fragments F1+2, von weniger als 20 %. Auch konnte keine Korrelation zwischen der Schwere der thromboembolischen Erkrankung bzw. des thrombotischen Ereignisses und der Höhe des Blutspiegels dieses und der anderen molekularen Marker gefunden werden.

Aus den Erfahrungen mit derartig betroffenen kranken Menschen ist abzuleiten, daß es in der Hämostaseologie bisher kein molekulares Meßprinzip gibt, das durch die Bestimmung des aktuellen Blutspiegels des Markers Rückschlüsse auf die Intensität der Gerinnungsaktivierung zuläßt. Als Ursache hierfür kommt in Frage, daß die molekularen Marker als körpereigene Stoffwechselprodukte der Gerinnungsenzyme mehr oder weniger schnell durch Klärmechanismen aus der Zirkulation entfernt werden. Dabei ist zu berücksichtigen, daß die Marker abhängig von der erhaltenen Organfunktion mehr oder weniger rasch metabolisiert werden, vor allem im Bereich des Leberstoffwechsels.

Die JP-A-63209587 offenbart die Herstellung eines an Dextran gekoppelten Enzyminhibitors (gegen Trypsin und Chymotrypsin) und dessen Verwendung als in vivo Diagnostikum zur Analyse der Enzymaktivität.

Der Erfindung liegt somit die Aufgabe zugrunde, sensible Marker für die Erfassung einer frühen Phase einer Gerinnungsaktivierung aufzufinden. Der molekulare Marker soll weiterhin eine breite diagnostische Bandbreite zur Erfassung von intravasalen Aktivierungsreaktionen bei der Blutgerinnung besitzen. Dabei soll der Marker unabhängig von Stoffwechselvorgängen bzw. Klärungsreaktionen im Organismus wirken. Es ist zu gewährleisten, daß sich der Marker rasch ausschließlich im Blutkreislauf verteilt, nicht oder nur in geringem Maße metabolisiert wird und nur langsam eliminiert wird.

Erfindungsgemäß wird diese Aufgabe durch die Verwendung eines an einen großmolekularen Träger gebundenen Inhibitors eines Aktivierungsprodukts der Blutgerinnungskaskade oder eines aktivierten Enzyms der Fibrinolyse als molekularer Marker zur Herstellung eines Präparats zur Bestimmung einer Aktivierung dieses Enzyms zur Diagnostik des Enzyms gelöst.

Es wurde nun überraschenderweise gefunden, daß an einen großmolekularen Träger gebundene Hemmstoffe eines aktivierbaren Stoffwechselenzyms, d.h. komplex gebundene Hemmstoffe eines Aktivierungsprodukts der Blutgerinnungskaskade oder eines aktivierten Enzyms der Fibrinolyse, sich ausschließlich und rasch im Blutkreislauf verteilen, im Organismus nur langsam abgebaut oder ausgeschieden werden und noch nahezu dieselbe Affinität zu dem Enzym haben wie die freien ungebundenen Hemmstoffe. Dieses Prinzip ist auf alle aktivierbaren Schlüsselenzyme anwendbar, für die hochaffine und spezifische Inhibitoren zur Verfügung stehen. Es eignet sich insbesondere für die Aktivierungsprodukte der Gerinnungskaskade, wie z.B. Thrombin, aktivierter Faktor VII oder aktivierter Faktor X, aber auch für die aktivierten Enzyme der Fibrinolyse, z.B. Gewebs-Plasminogen-Aktivator (tPA) oder Plasmin.

Die nachstehende Beschreibung bezieht sich auf eine bevorzugte Ausführungsform der Erfindung, d.h. die Verwendung von Thrombininhibitoren. Es ist jedoch zu verstehen, daß das an diesem Beispiel dargestellte molekulare Markerprinzip für diagnostische Überwachungen im Blut entsprechend auf jede beliebige Kombination aus einem aktivierbaren Enzym und einem hochaffinen und spezifischen Inhibitor gemäß Anspruch 1, der an einen hochmolekularen Trägerstoff gebunden ist, angewendet werden kann.

Es wurde überraschenderweise gefunden, daß an großmolekulare Träger gebundene Thrombininhibitoren (komplex gebundene Thrombininhibitoren) sich ausschließlich und rasch im Blutkreislauf verteilen, im Organismus nur langsam abgebaut bzw. eliminiert werden und noch immer die gleiche Affinität zu Thrombin haben wie die freien Thrombininhibitoren und sich dadurch für diagnostische Zwecke eignen. Der Einsatz von ungebundenen Thrombininhibitoren für derartige Diagnostika ist nicht möglich, weil sich diese Stoffe im gesamten Körper, nicht nur im Blut, verteilen und bei einem Verbrauch umverteilt werden ohne erkennbaren Abfall der Konzentration im Blut.

Als Thrombininhibitoren können hochaffine, natürliche Thrombininhibitoren, z.B. Hirudin, aber auch alle anderen direkt bindenden synthetischen Thrombininhibitoren mit hoher Affinität zu Thrombin verwendet werden. Beispiele hierfür sind PEG-gebundene 4-Amidinophenylalanine (vgl. Peptide Research, 8, Nr. 2, 78-85 (1995). Als hochmolekulare Träger können natürliche oder synthetische Substanzen verwendet werden, beispielsweise Polyethylenglykol, Dextran, aber auch körpereigene Blutproteine. Weitere Beispiele hierfür sind Albumin, γ-Globuline, aber auch Ferritin, succinylierte Gelatine, vernetzte Polypeptide und Polyhydroxystärke.

Zur Anwendung eignet sich ein Dextran-gebundenes (DP) Hirudin, aber auch an Polyethylenglykol (PEG) gekoppeltes Hirudin, bzw. Hirudin, das an humane körpereigene Eiweiße gebunden wurde. Aufgrund ihrer Molekülgröße unterliegen diese Proteine (Albumin-gebundenes Hirudin bzw. an bestimmte Gammaglobuline gebundene Hirudine) nur einem sehr langsamen biologischen Klärungsprozeß.

Derartige Komplex-gebundene Hirudine haben den großen Vorteil, daß sie sich nahezu ausschließlich in dem Blut verteilen und keine Extravasation in den extrazellulären Flüssigkeitsraum haben. Dadurch können bereits geringe Mengen dieses Markers, wenn im Blut verteilt, als hochaffiner, schnell und stark bindender Inhibitor für intravasal aktiviertes Thrombin bzw. aktivierte Intermediate der Prothrombin-Thrombin-Umwandlung fungieren. Durch die Bindung des aktivierten Enzyms an den Komplex-gebundenen Thrombininhibitor vermindert sich die Menge des "freien" komplexierten Hirudins in dem Ausmaß, wie aktives Thrombin in der Zirkulation verfügbar wird.

Hirudin, auch an Makromoleküle gebundenes Hirudin, hat eine hohe Affinität zur Serinprotease Thrombin. Die singuläre Spezifität ausschließlich für Thrombin-Spezies läßt diesen Marker nur für diese Serinprotease im Organismus wirksam werden. Bindungen an andere Enzyme sind nicht möglich und nicht bekannt.

Wird im Organismus durch eine permanente Gerinnungsaktivierung als Endprodukt der intrinsischen oder extrinsischen Gerinnung das Schlüsselenzym des Gerinnungssystems, die Serinprotease Thrombin im Blut verfügbar, wird diese sofort von dem im Blut vorhandenen molekularen Marker CBH gebunden und inaktiviert. Die Menge des Komplex-gebundenen Hirudins, des freien Inhibitors, vermindert sich in dem Maße im strömenden Blut, wie Thrombin-Hirudin-Komplexe entstehen.

Der "freie" molekulare Marker, Komplex-gebundenes Hirudin, wird mit Hilfe einer empfindlichen, spezifischen und rasch durchführbaren Nachweismethode für freies Hirudin erfaßt. Durch ein wiederholtes Monitoring lassen sich auch diskrete Gerinnungsaktivierungen mit Hilfe dieser Nachweismethode im Organismus zu einem frühen Zeitpunkt erkennen und quantifizieren.

Die erfindungsgemäßen molekularen Marker werden in einer Dosierung von 0,005 - 0,5 mg/kg, bevorzugt 0,01 - 0,05 mg/kg, am bevorzugtesten 0,01 - 0,02 mg/kg, bezogen auf das Körpergewicht der Patienten, appliziert. Sie werden parenteral, bevorzugt intravenös, verabreicht. Daneben ist auch eine orale Verabreichung, bei der durch entsprechende Konfektionierung eine Resorption des Markers sichergestellt ist, möglich. Dazu werden die erfindungsgemäßen molekularen Marker zusammen mit üblichen Hilfs- und Trägerstoffen in eine für diese Verabreichung geeignete Formulierung gebracht. So können beispielsweise Komplex-gebundene Hirudinpräparate entweder in einer gefriergetrockneten Formulierung (zu lösen in 5 ml Aqua; PEG-Hirudin, Albumin-Hirudin, γ-Globulin-Hirudin) oder in gebrauchsfertiger Injektionslösung (z.B. 5 ml; Gelatine-Hirudin, Hydroxystärke-Hirudin) hergestellt werden, wobei der Gehalt an Hirudin zweckdienlicherweise 5 mg Hirudin/Ampulle beträgt. Zur Anwendung wird die benötigte Dosis nach der Formel

$$\text{kg Körpermasse} : 20 \hat{=} \text{ml Applikationsvolumen}$$

berechnet und als i.v. Bolus appliziert.

Die Erfindung wird anhand des folgenden Beispiels näher erläutert.

Beispiel: Komplex-gebundenes Hirudin als molekularer Marker

- Patienten aus der Risikogruppe erhalten eine ihrem Körpergewicht entsprechende Dosis Komplex-gebundenes Hirudin intravenös appliziert. Nach kurzer Verteilungszeit (10 - 15 min) stellt sich ein konstanter Blutspiegel dieses CBH ein. Durch entsprechende Kontrolluntersuchungen an freiwilligen gesunden Probanden ist die Eliminationshalbwertszeit für CBH als Vergleichsgröße bekannt.

- Bei den Thrombose-gefährdeten Patienten werden in kürzeren Zeitabständen kleine Mengen von citriertem Vollblut (0,5 ml) gewonnen. Mit Hilfe einer spezifischen Nachweismethode für Hirudin, der Ecarin Clotting Time (ECT, Europ. Patent Nr. 93 903 232.2), wird das freie zirkulierende CBH erfaßt. Die Ecarin Clotting Time ist eine Aktivitätsbestimmungsmethode und erfaßt äußerst empfindlich freies Hirudin, jedoch nicht Hirudin-Thrombin-Komplexe. Der Abfall des Blutspiegels von CBH steht in direkter Korrelation zur Stärke der Thrombinliberation in der Blutzirkulation. Durch die Möglichkeit der mathematischen Modellierung der "Verschwindens-Rate" bzw. der stärkeren Abnahme des freien CBH-Blutspiegels ist eine zeitliche Quantifizierung der Thrombinliberation in der Blutbahn gegeben. Durch diese Methode ist es möglich, auch frühe Phasen der Gerinnungsaktivierung, die bisher diagnostisch nicht erreichbar waren, zu erfassen.

In den weiteren Ausführungen werden diese molekularen Thrombin-"Sonden" in einer tierexperimentellen Modellierung auf ihre Effizienz hin überprüft.

Die hierbei benutzten Versuchstiere waren Ratten (HAN-WISt, Zentrale Versuchstierhaltung der Universität Jena) und Kaninchen (Chinchilla-Bastards, Savo, Bad Kislegg). Von beiden Spezies wurden sowohl weibliche als auch männliche Tiere, SPF-Haltungsstandard, verwendet. Die Ratten wurden mit Ethylurethan (1,5 g/kg subkutan), die Kaninchen mit Pentobarbital (25 mg/kg i. v.) narkotisiert. Als Bestimmungsmethode für Hirudine im Blut wurden zwei unterschiedliche Detektionsmethoden benutzt. Neben der Ecarin-Clotting-Time wurde auch eine chromogene Substratmethode verwendet. Hierbei wurde dem Plasma (1:10 verdünnt) chromogenes Substrat (Chromozym TM, Pentapharm Basel) und Ecarin (25 EU/ml) bzw. Thrombin (1,5 NIH-U/ml) zugegeben und nach 2 min die Extinktion im Spektralphotometer gemessen (NIH-U: internationaler Standard für die Thrombin-Gerinnungsaktivität; NIH = National Institute of Health).

Das Komplex-gebundene Hirudin wurde in zwei verschiedenen Hirudin-Präparationen benutzt:

1. Dextran-Hirudin (Dextran 150 kDa) wurde mittels einer Methode nach Walsmann et al. an Hirudin gebunden. Die spezifische Aktivität betrug 971 ATU/mg (ATU = Antithrombin-Units = internationaler Standard für direkte Thrombininhibitoren).

2. PEG-gekoppeltes Hirudin. Zur Verwendung kamen kommerzielle Präparationen der Fa. Knoll (PEG-Hirudin/ 144 bzw. PEG/153).

1. Einfluß von Thrombin auf den Plasma-Spiegel von PEG-Hirudin bei Ratten

Versuchsdesign:

Narkotisierten Ratten wurde PEG-Hirudin in einer Dosierung von 140 ATU/kg Körpergewicht als Bolus intravenös appliziert (ATU = Antithrombin-Units). 10 min später erhielten die Ratten eine Infusion von Thrombin bzw. Kontrolltiere das gleiche Infusionsvolumen Kochsalzlösung. In 60-min-Abständen wurden den Ratten 0,5 ml Citratblut über einen Verweilkatheter in der V. jugulans entnommen, um damit die Blutspiegelkontrollen des molekularen Markers durchzuführen. Thrombin wurde den Ratten in folgenden Konzentrationen über 360 min infundiert: 35, 70, 140, 250 und 500 $NIH-U/kg \times h^{-1}$. Die Ergebnisse sind in der Abb. 1 dargestellt. Es ist zu erkennen, daß nach initialer kurzer Verteilungszeit ein relativ konstanter Blutspiegel an PEG-Hirudin im Plasma nachweisbar ist. Die approximative Halbwertszeit liegt bei etwa 12-14 h. Bei der Infusion von 35 $NIH-U/kg \times h^{-1}$ Thrombin ist der Blutspiegelverlauf des PEG-Hirudins mit dem in der Kontrollgruppe (Kochsalzinfusion) nahezu identisch. Bei 70 $NIH-U/kg \times h^{-1}$ Thrombin ist bereits nach 120 min ein deutlich beschleunigter Blutspiegelabfall des PEG-Hirudins nachweisbar. Bei höheren Thrombin-Dosen verschwindet das freie PEG-Hirudin sehr rasch aus der Blutzirkulation. Bei 250 bzw. 500 NIH-U Thrombin ist bereits nach 120 min kein PEG-Hirudin im Plasma mehr nachweisbar.

**Abb. 1: Einfluß einer kontinuierlichen Thrombininfusion auf den Plasmaspiegel von PEG-Hirudin/144 (140 U/kg)**

2. Einfluß von wiederholter Thrombin-Applikation auf den PEG-Hirudinspiegel von Ratten

Das bei diesen Versuchen benutzte PEG-Hirudin (PEG153) ergab einen etwa gleichen Verteilungs- und Blutspiegelverlauf wie im vorher dargestellten Versuchsbeispiel. Nach jeweils 15-minütiger Kurzinfusion von 100 NIH-U/ kg Thrombin nach 120, 180 und 240 min ist nur eine diskrete Beeinflussung des PEG-Hirudinspiegels nachweisbar, dagegen ist bei Applikation von jeweils 250 NIH-U/kg Thrombin nach 3 h ein stärkerer Abfall des PEG-Hirudin-Blutspiegels aufgetreten, der bei einer Dosissteigerung auf 500 NIH-U/kg Thrombin noch größer ist.

**Abb. 2: Einfluß von kurzen und wiederholten Thrombininfusionen (3 x 20 min) auf den Plasmaspiegel von PEG-Hirudin/153 (150 U/kg)**

3. Einfluß von Thrombokinase auf den Plasmaspiegel von Dextran-Hirudin bei Kaninchen

Die Kaninchen wurden mit Dextran-Hirudin 5000 ATU/kg vorbehandelt. Bei der messenden Verfolgung des Dextran-Hirudin-Blutspiegels ist zu erkennen, daß bei den Kaninchen erst nach 24 h ein konstanter Hirudin-Blutspiegel nachweisbar ist. Bei Infusion einer gereinigten Thrombokinaselösung (1 ml/kg/h) über 6 h ist ein stärkerer Abfall des Dextran-Hirudin-Blutspiegels nachweisbar. Für diese Untersuchungen wurden die Resultate von 5 Einzelversuchen zusammengefaßt.

Abb. 3: Einfluß einer Thrombokinaseinfusion auf den Blutspiegel von Dextran-Hirudin

Aus diesen Untersuchungen ergibt sich, daß Dextran-Hirudin bei Kaninchen aufgrund von Interaktionen des Dextrans mit Oberflächenstrukturen der Endothelzellen, des RES der Leber und der korpuskulären Bestandteile des Blutes eine sehr lange Verteilungsphase hat (24 h). Dextran-Hirudin eignet sich für Marker-Untersuchungen bei dieser Tierspezies nur bedingt. Es war bei den Untersuchungen nicht zu erkennen, ob sich das Dextran-Hirudin auch in tiefere Kompartimente des Kaninchen-Organismus verteilte. Die PEG-gekoppelten Hirudine (PEG144 bzw. 153) dagegen erwiesen sich in den hier dargestellten Versuchen an Ratten für eine entsprechende molekulare Marker-Modellierung als geeignet. Bereits nach 10 min war ein relativ konstantes Verteilungsgleichgewicht in der Zirkulation der Ratten erreicht, und die Modellierung einer intravasalen Gerinnungsaktivierung mittels kontinuierlicher Infusion von kleinen Mengen Thrombin bzw. diskontinuierlicher Applikation von Thrombin konnte durch eine entsprechende Störung des PEG-Hirudin-Blutspiegels messend verfolgt werden.

Aus diesen tierexperimentellen Modell-Untersuchungen kann abgeleitet werden, daß sich Komplex-gebundene Hirudine als molekulare Marker für eine intravasale Gerinnungsaktivierung eignen. Der Vorteil dieser hier dargestellten molekularen Marker ist darin zu sehen, daß derartige großmolekulare Hirudinkomplexe keinerlei Klärfunktion im Organismus unterliegen. Sie sind permanent verfügbar für die Bindung von aktivem Thrombin, welches als Aktivierungsendprodukt der Gerinnungsaktivierung in der Zirkulation permanent auftreten kann. Durch eine empfindliche Detektionsmethode des freien Markerspiegels ist eine definitive Gerinnungsaktivierung quantifizierbar.

**Patentansprüche**

1.   Verwendung eines an einen großmolekularen Träger gebundenen Inhibitors eines Aktivierungsprodukts der Blutgerinnungskaskade oder eines aktivierten Enzyms der Fibrinolyse als molekularer Marker zur Herstellung eines Präparats zur Bestimmung einer Aktivierung dieses Enzyms zur Diagnostik des Enzyms.

2.   Verwendung nach Anspruch 1, **dadurch gekennzeichnet, daß** der großmolekulare Träger Dextran, Polyethylenglykol (PEG) oder ein körpereigenes Eiweiß ist.

**3.** Verwendung nach einem der Ansprüche 1 oder 2, **dadurch gekennzeichnet, daß** der an einen großmolekularen Träger gebundene Inhibitor ein Thrombininhibitor ist und als molekularer Marker zur Herstellung eines Präparats zur Bestimmung einer Gerinnungsaktivierung bei der Gerinnungsdiagnostik und Therapieüberwachung verwendet wird.

**4.** Verwendung nach Anspruch 3, **dadurch gekennzeichnet, daß** der Thrombininhibitor Hirudin ist.

**5.** Verwendung nach Anspruch 3, **dadurch gekennzeichnet, daß** der an einen großmolekularen Träger gebundene Thrombininhibitor Dextran-Hirudin oder PEG-gekoppeltes Hirudin ist.

**6.** Verwendung nach einem der Ansprüche 3 bis 5, **dadurch gekennzeichnet, daß** der an einen großmolekularen Träger gebundene Thrombininhibitor in einer Dosierung von 0,005 - 0,5 mg/kg, bevorzugt 0,01 - 0,05 mg/kg, am bevorzugtesten 0,01 - 0,02 mg/kg eingesetzt wird.

**7.** Verwendung nach einem der Ansprüche 3 bis 6, **dadurch gekennzeichnet, daß** der an einen großmolekularen Träger gebundene Thrombininhibitor parenteral verabreicht wird.

**8.** Verwendung nach Anspruch 1, **dadurch gekennzeichnet, daß** der an einen großmolekularen Träger gebundene Inhibitor ein Plasmininhibitor oder Gewebs-Plasminogen-Aktivator-Inhibitor ist und als molekularer Marker zur Herstellung eines Präparats zur Bestimmung der Fibrinolyseaktivierung verwendet wird.

**Claims**

**1.** Use of an inhibitor, bound to a high molecular weight support, of an activation product of the blood clotting cascade or of an activated fibrinolysis enzyme as a molecular marker for the production of a preparation for determining activation of this enzyme for enzyme diagnostic purposes.

**2.** Use according to claim 1, **characterised in that** the high molecular weight support is dextran, polyethylene glycol (PEG) or an endogenous protein.

**3.** Use according to one of claims 1 or 2, **characterised in that** the inhibitor bound to a high molecular weight support is a thrombin inhibitor and is used as a molecular marker for the production of a preparation for determining activation of clotting in clotting diagnostics and therapeutic monitoring.

**4.** Use according to claim 3, **characterised in that** the thrombin inhibitor is hirudin.

**5.** Use according to claim 3, **characterised in that** the thrombin inhibitor bound to a high molecular weight support is dextran-hirudin or PEG-coupled hirudin.

**6.** Use according to one of claims 3 to 5, **characterised in that** the thrombin inhibitor bound to a high molecular weight marker is used at a dosage of 0.005-0.5 mg/kg, preferably of 0.01-0.05 mg/kg, most preferably of 0.01-0.02 mg/kg.

**7.** Use according to one of claims 3 to 6, **characterised in that** the thrombin inhibitor bound to a high molecular weight support is administered parenterally.

**8.** Use according to claim 1, **characterised in that** the inhibitor bound to a high molecular weight support is a plasmin inhibitor or a tissue plasminogen activator inhibitor and is used as a molecular marker for the production of a preparation for determining activation of fibrinolysis.

**Revendications**

**1.** Utilisation d'un inhibiteur, lié à un support de poids moléculaire élevé, d'un produit d'activation des réactions en cascade de la coagulation sanguine ou d'une enzyme activée de la fibrinolyse en tant que marqueur moléculaire pour la production d'une préparation pour déterminer une activation de cette enzyme pour le diagnostic de l'enzyme.

**2.** Utilisation selon la revendication 1, **caractérisée en ce que** le support de poids moléculaire élevé est du dextrane, un polyéthylèneglycol (PEG) ou de l'albumine propre au corps.

**3.** Utilisation selon l'une des revendications 1 et 2, **caractérisée en ce que** l'inhibiteur lié à un support de poids moléculaire élevé est un inhibiteur de la thrombine et est utilisé en tant que marqueur moléculaire pour la production d'une préparation pour déterminer une activation de la coagulation lors d'un diagnostic de coagulation et d'une surveillance thérapeutique.

**4.** Utilisation selon la revendication 3, **caractérisée en ce que** l'inhibiteur de la thrombine est l'hirudine.

**5.** Utilisation selon la revendication 3, **caractérisée en ce que** l'inhibiteur de la thrombine lié à un support de poids moléculaire élevé est du dextrane-hirudine ou de l'hirudine couplée avec un PEG.

**6.** Utilisation selon l'une des revendications 3 à 5, **caractérisée en ce qu'**on utilise l'inhibiteur de la thrombine lié à un support de poids moléculaire élevé à une dose de 0,005 à 0,5 mg/kg, de préférence de 0,01 à 0,05 mg/kg et de la façon la plus préférée de 0,01 à 0,02 mg/kg.

**7.** Utilisation selon l'une des revendications 3 à 6, **caractérisée en ce que** l'inhibiteur de la thrombine lié à un support de poids moléculaire élevé est administré par voie parentérale.

**8.** Utilisation selon la revendication 1, **caractérisée en ce que** l'inhibiteur lié à un support de poids moléculaire élevé est un inhibiteur de la plasmine ou un inhibiteur de l'activateur du plasminogène tissulaire, et on l'utilise en tant que marqueur moléculaire pour la production d'une préparation pour la détermination de l'activation de la fibrino-lyse.